(19) Europäisches Patentamt
European Patent Office
Office européen des brevets

(11) EP 2 155 700 B1

(12) EUROPEAN PATENT SPECIFICATION

(45) Date of publication and mention of the grant of the patent:
**06.10.2010 Bulletin 2010/40**

(21) Application number: **08736619.1**

(22) Date of filing: **29.04.2008**

(51) Int Cl.:
***C07D 257/04*** *(2006.01)* ***A61K 31/41*** *(2006.01)*
***A61P 9/00*** *(2006.01)*

(86) International application number:
**PCT/EP2008/055216**

(87) International publication number:
**WO 2008/135448 (13.11.2008 Gazette 2008/46)**

(54) **BETA-KETO-AMIDE DERIVATIVES USEFUL AS ION CHANNEL MODULATORS**

ALS IONENKANALMODULATOREN GEEIGNETE BETA-KETOAMIDDERIVATE

NOUVEAUX DÉRIVÉS DE BÉTA-CÉTO-AMIDE SERVANT DE MODULATEURS DE CANAUX IONIQUES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **03.05.2007 DK 200700669**
**04.05.2007 US 916117 P**

(43) Date of publication of application:
**24.02.2010 Bulletin 2010/08**

(73) Proprietor: **NeuroSearch A/S**
**2750 Ballerup (DK)**

(72) Inventors:
- **NARDI, Antonio**
**DK-2750 Ballerup (DK)**
- **CHRISTENSEN, Jeppe, Kejser**
**DK-2750 Ballerup (DK)**
- **GRUNNET, Morten**
**DK-2750 Ballerup (DK)**
- **CHRISTOPHERSEN, Palle**
**DK-2750 Ballerup (DK)**
- **JONES, David, Spencer**
**DK-2750 Ballerup (DK)**
- **NIELSEN, Elsebet, Østergaard**
**DK-2750 Ballerup (DK)**
- **STRØBÆK, Dorte**
**DK-2750 Ballerup (DK)**
- **MADSEN, Lars, Siim**
**DK-2750 Ballerup (DK)**

(56) References cited:
**WO-A-01/01981** **WO-A-2004/009533**
**WO-A-2005/085221**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 155 700 B1

## Description

### TECHNICAL FIELD

This invention relates to novel β-keto-amide derivatives that are found to be potent modulators of ion channels, and in particular potassium channels and chloride channels, and, as such, are valuable candidates for the treatment of diseases or disorders as diverse as those which are responsive to the modulation of potassium channels.

### BACKGROUND ART

Ion channels are cellular proteins that regulate the flow of ions through cellular membranes of all cells and are classified by their selective permeability to the different of ions (potassium, chloride, sodium etc.). Potassium channels, which represent the largest and most diverse sub-group of ion channels, selectively pass potassium ions and, doing so, they principally regulate the resting membrane potential of the cell and/or modulate their level of excitation.

Dysfunction of potassium channels, as well as other ion channels, generates loss of cellular control resulting in altered physiological functioning and disease conditions. Ion channel blockers and openers, by their ability to modulate ion channel function and/ or regain ion channel activity in acquired or inherited channelopathies, are being used in the pharmacological treatment of a wide range of pathological diseases and have the potential to address an even wider variety of therapeutic indications. For instance, the primary indications for potassium channel openers encompass conditions as diverse as diabetes, arterial hypertension, cardiovascular diseases, urinary incontinence, atrial fibrillation, epilepsy, pain, and cancer.

Among the large number of potassium channel types, the large-conductance calcium-activated potassium channel subtype is an obvious site for pharmacological intervention and for the development of new potassium channel modulators. Their physiological role has been especially studied in the nervous system, where they are key regulators of neuronal excitability and of neurotransmitter release, and in smooth muscle, where they are crucial in modulating the tone of vascular, broncho-tracheal, urethral, uterine or gastro-intestinal musculature.

Given these implications, small agents with BK-opening properties could have a potentially powerful influence in the modulation and control of numerous consequences of muscular and neuronal hyperexcitability, such as asthma, urinary incontinence and bladder spasm, gastroenteric hypermotility, psychoses, post-stroke neuroprotection, convulsions, anxiety and pain. As far as the cardiovascular system is concerned, the physiological function of these ion channels represents a fundamental steady state mechanism, modulating vessel depolarisation, vasoconstriction and increases of intravascular pressure, and the development of selective activators of BK channels is seen as a potential pharmacotherapy of vascular diseases, including hypertension, erectile dysfunction, coronary diseases and vascular complications associated with diabetes or hypercholesterolemia.

Chloride channels serve a wide variety of specific cellular functions and contribute to the normal function of i.a. skeletal and smooth muscle cells. Chloride channels are probably found in every cell, from bacteria to mammals. Their physiological tasks range from cell volume regulation to stabilization of the membrane potential, transepithelial or transcellular transport and acidification of intracellular organelles.

### SUMMARY OF THE INVENTION

It is an object of the invention to provide novel β-keto-amide derivatives useful as ion channel modulators. The β-keto-amide derivatives of the invention may be characterised by Formula I

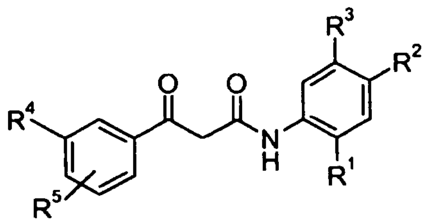

(I)

a stereoisomer or a mixture of its stereoisomers, or a pharmaceutically-acceptable addition salt thereof, wherein
$R^1$ represents a tetrazolyl or a 5-oxo-4,5-dihydro-[1,2,4]oxadiazol-3-yl group;

$R^2$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and $R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy; or
$R^2$ represents hydrogen; and $R^3$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and
$R^4$ and $R^5$, independently of each other, represent halo, trifluoromethyl, trifluoromethoxy or phenyl.

In another aspect the invention provides pharmaceutical compositions comprising a therapeutically effective amount of a β-keto-amide derivative of the invention.

In a third aspect the invention relates to the use of the β-keto-amide derivatives of the invention for the manufacture of pharmaceutical compositions.

In a further aspect the invention provides a method of treatment, prevention or alleviation of a disease or a disorder or a condition of a living animal body, including a human, which disorder, disease or condition is responsive to modulation of ion channels, which method comprises the step of administering to such a living animal body in need thereof, a therapeutically effective amount of the β-keto-amide derivative of the invention.

Other objects of the invention will be apparent to the person skilled in the art from the following detailed description and examples.

## DETAILED DISCLOSURE OF THE INVENTION

In its first aspect the invention provides novel β-keto-amide derivatives of Formula I

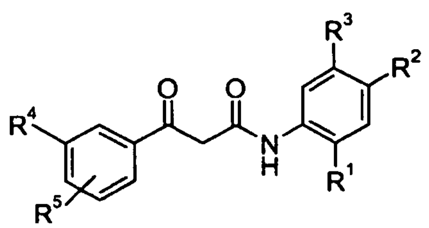

(I)

a stereoisomer or a mixture of its stereoisomers, or a pharmaceutically-acceptable addition salt thereof, wherein
$R^1$ represents a tetrazolyl or a 5-oxo-4,5-dihydro-[1,2,4]oxadiazol-3-yl group;
$R^2$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and $R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy; or
$R^2$ represents hydrogen; and $R^3$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and
$R^4$ and $R^5$, independently of each other, represent halo, trifluoromethyl, trifluoromethoxy or phenyl.

In a more preferred embodiment the β-keto-amide derivative of the invention is a compound of Formula Ia

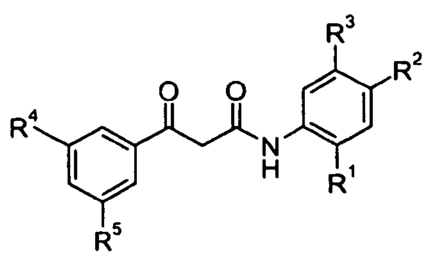

(Ia)

a stereoisomer or a mixture of its stereoisomers, or a pharmaceutically-acceptable addition salt thereof, wherein $R^1$,

$R^2$, $R^3$, $R^4$ and $R^5$ are as defined above.

In another more preferred embodiment the β-keto-amide derivative of the invention is a compound of Formula Ib

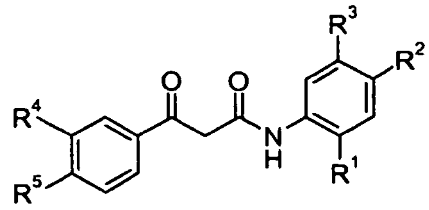

**(Ib)**

a stereoisomer or a mixture of its stereoisomers, or a pharmaceutically-acceptable addition salt thereof, wherein $R^1$, $R^2$, $R^3$, $R^4$ and $R^5$ are as defined above.

In a third more preferred embodiment the β-keto-amide derivative of the invention is a compound of Formula Ib, wherein
$R^1$, $R^2$ and $R^3$ are as defined above;
$R^4$ represents trifluoromethyl or trifluoromethoxy; and
$R^5$ represent halo, in particular chloro.

In an even more preferred embodiment the β-keto-amide derivative of the invention is a compound of Formula Ib, wherein
$R^1$, $R^2$ and $R^3$ are as defined above;
$R^4$ represents trifluoromethyl; and
$R^5$ represent halo, in particular chloro.

In another more preferred embodiment the β-keto-amide derivative of the invention is a compound of Formula I, Ia or Ib, or a pharmaceutically-acceptable addition salt thereof, wherein $R^1$ represents a tetrazolyl or a 5-oxo-4,5-dihydro-[1,2,4]oxadiazol-3-yl group.

In a more preferred embodiment $R^1$ represents a tetrazolyl group.

In another more preferred embodiment $R^1$ represents a 5-oxo-4,5-dihydro-[1,2,4]oxadiazol-3-yl group.

In a third more preferred embodiment the β-keto-amide derivative of the invention is a compound of Formula I, Ia or Ib, or a pharmaceutically-acceptable addition salt thereof, wherein
$R^2$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and $R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy; or
$R^2$ represents hydrogen; and $R^3$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy.

In a fourth more preferred embodiment the β-keto-amide derivative of the invention is a compound of Formula I, Ia or Ib, or a pharmaceutically-acceptable addition salt thereof, wherein
$R^2$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and
$R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy.

In an even more preferred embodiment $R^2$ represents halo or phenyl, which phenyl is optionally substituted with halo, in particular chloro, trifluoromethyl or trifluoromethoxy.

In a still more preferred embodiment $R^2$ represents halo, in particular bromo.

In another more preferred embodiment $R^2$ represents phenyl, which phenyl is optionally substituted with halo, in particular chloro, trifluoromethyl or trifluoromethoxy.

In an even more preferred embodiment $R^2$ represents phenyl substituted with halo, in particular chloro, trifluoromethyl, or trifluoromethoxy.

In a still more preferred embodiment $R^2$ represents phenyl substituted with halo, in particular chloro, or trifluoromethoxy.

In another even more preferred embodiment $R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy.

In a still more preferred embodiment $R^3$ represents hydrogen or halo, in particular chloro.

In a yet more preferred embodiment $R^3$ represents hydrogen.

In a third even more preferred embodiment $R^3$ represents halo, in particular chloro.

In a fifth more preferred embodiment the β-keto-amide derivative of the invention is a compound of Formula I, Ia or Ib, or a pharmaceutically-acceptable addition salt thereof, wherein
$R^2$ represents hydrogen; and
$R^3$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy.

In a still more preferred embodiment $R^3$ represents halo, in particular chloro.

In a sixth more preferred embodiment the β-keto-amide derivative of the invention is a compound of Formula I, Ia or Ib, or a pharmaceutically-acceptable addition salt thereof, wherein $R^4$ and $R^5$, independently of each other, represent halo or trifluoromethyl or trifluoromethoxy.

In an even more preferred embodiment $R^4$ and $R^5$ both represent halo, in particular chloro, trifluoromethyl or trifluoromethoxy.

In a yet more preferred embodiment $R^4$ and $R^5$ both represent trifluoromethyl.

In another even more preferred embodiment
$R^4$ represents trifluoromethyl or trifluoromethoxy; and
$R^5$ represent halo, in particular chloro.

In a yet more preferred embodiment
$R^4$ represents trifluoromethyl; and
$R^5$ represent halo, in particular chloro.

In a most preferred embodiment the β-keto-amide derivative of the invention is
3-(3,5-Bis-trifluoromethyl-phenyl)-*N*-[4-bromo-2-(1*H*-tetrazol-5-yl)-phenyl]-3-oxo-propionamide;
3-(3,5-Bis-trifluoromethyl-phenyl)-*N*-[4'-chloro-3-(1*H*-tetrazol-5-yl)-biphenyl-4-yl]-3-oxo-propionamide;
3-(3,5-Bis-trifluoromethyl-phenyl)-3-oxo-*N*-[3-(1*H*-tetrazol-5-yl)-4'-trifluoromethoxy-biphenyl-4-yl]-propionamide; or
*N*-[5-Chloro-2-(1*H*-tetrazol-5-yl)-phenyl]-3-(4-chloro-3-trifluoromethylphenyl)-3-oxo-propionamide;
or a pharmaceutically-acceptable addition salt thereof.

Any combination of two or more of the embodiments described herein is considered within the scope of the present invention.

Definition of Substituents

In the context of this invention halo represents fluoro, chloro, bromo or iodo.

Pharmaceutically Acceptable Salts

The β-keto-amide derivatives of the invention may be provided in any form suitable for the intended administration. Suitable forms include pharmaceutically (i.e. physiologically) acceptable salts, and pre- or prodrug forms of the β-keto-amide derivative of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Examples of pharmaceutically acceptable cationic salts of a β-keto-amide derivative of the invention include, without limitation, the sodium, the potassium, the calcium, the magnesium, the lithium, and the ammonium salt, and the like, of a β-keto-amide derivative of the invention containing an anionic group. Such cationic salts may be formed by procedures well known and described in the art.

**Steric Isomers**

It will be appreciated by those skilled in the art that the compounds of the present invention may exist in different stereoisomeric forms, including enantiomers. diastereomers, as well as geometric isomers (cis-trans isomers). The invention includes all such isomers and any mixtures thereof including racemic mixtures.

Racemic forms can be resolved into the optical antipodes by known methods and techniques. One way of resolving racemates into the optical antipodes is based upon chromatography on an optical active matrix. Racemic

compounds of the present invention can thus be resolved into their optical antipodes, e.g., by fractional crystallisation of D- or L- (tartrates, mandelates, or camphorsulphonate) salts for example.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optically active starting materials or intermediates.

### Methods of Preparation

The compounds according to the invention may be prepared by conventional methods for chemical synthesis, e.g. those described in the working examples.

### Biological Activity

The β-keto-amide derivatives of the invention have been found to possess ion channel modulating activity, and in particular potassium channel activating activity and chloride channels blocking activity, as measured by standard electrophysiological methods. Due to their activity at the potassium channels and chloride channels, the β-keto-amide derivatives of the invention are considered useful for the treatment of a wide range of diseases and conditions.

In a special embodiment, the β-keto-amide derivatives of the invention are considered useful for the treatment, prevention or alleviation of a respiratory disease, epilepsy, convulsions, seizures, absence seizures, vascular spasms, coronary artery spasms, motor neuron diseases, myokymia, renal disorders, polycystic kidney disease, bladder hyperexcitability, bladder spasms, urinogenital disorders, urinary incontinence, bladder outflow obstruction, erectile dysfunction, gastrointestinal dysfunction, gastrointestinal hypomotility disorders, gastrointestinal motility insufficiency, postoperative ileus, constipation, gastroesophageal reflux disorder, secretory diarrhoea, an obstructive or inflammatory airway disease, ischaemia, cerebral ischaemia, ischaemic heart disease, angina pectoris, coronary heart disease, ataxia, traumatic brain injury, stroke, Parkinson's disease, bipolar disorder, psychosis, schizophrenia, autism, anxiety, mood disorders, depression, manic depression, psychotic disorders, dementia, learning deficiencies, age related memory loss. memory and attention deficits, Alzheimer's disease, amyotrophic lateral sclerosis (ALS), dysmenorrhoea, narcolepsy, sleeping disorders, sleep apnoea, Reynaud's disease, intermittent claudication, Sjogren's syndrome, xerostomia, arrhythmia, cardiovascular disorders, hypertension, myotonic dystrophy, myotonic muscle dystrophia, spasticity, xerostomia, diabetes Type II, hyperinsulinemia, premature labour, cancer, brain tumours, inflammatory bowel disease, irritable bowel syndrome, colitis, colitis Crohn, immune suppression, hearing loss, migraine, pain, neuropathic pain, inflammatory pain, trigeminal neuralgia, vision loss, rhinorrhoea, ocular hypertension (glaucoma), baldness, cardiac arrhythmia, atrial arrhythmia, ventricular arrhythmia, atrial fibrillation, ventricular fibrillation, tachyarrhythmia, atrial tachyarrhythmia, ventricular tachyarrhythmia, bradyarrhythmia, or any other abnormal rhythm, e.g. caused by myocardial ischaemia, myocardial infarction, cardiac hypertrophy or cardiomyopathy.

In a more preferred embodiment, the β-keto-amide derivatives of the invention are considered useful for the treatment, prevention or alleviation of a respiratory disease, urinary incontinence, erectile dysfunction, anxiety, epilepsy, psychosis, schizophrenia, bipolar disorder, depression, amyotrophic lateral sclerosis (ALS), Parkinson's disease or pain.

In another more preferred embodiment, the β-keto-amide derivatives of the invention are considered useful for the treatment, prevention or alleviation of psychosis, schizophrenia, bipolar disorder, depression, epilepsy, Parkinson's disease or pain.

In a third more preferred embodiment, the β-keto-amide derivatives of the invention are considered useful for the treatment, prevention or alleviation of pain, mild or moderate or severe pain, pain of acute, chronic or recurrent character, pain caused by migraine, postoperative pain, phantom limb pain, inflammatory pain, neuropathic pain, chronic headache, central pain, pain related to diabetic neuropathy, to post therapeutic neuralgia, or to peripheral nerve injury.

In a fourth more preferred embodiment, the β-keto-amide derivatives of the invention are considered useful for the treatment, prevention or alleviation of cardiac arrhythmia, atrial arrhythmia, ventricular arrhythmia, atrial fibrillation, ventricular fibrillation, tachyarrhythmia, atrial tachyarrhythmia, ventricular tachyarrhythmia, bradyarrhythmia, or any other abnormal rhythm, e.g. caused by myocardial ischaemia, myocardial infarction, cardiac hypertrophy, cardiomyopathy or a genetic disease.

In a fifth more preferred embodiment, the β-keto-amide derivatives of the invention are considered useful for the treatment, prevention or alleviation of cardiac ischemia, ischemic heart disease, hypertrophic heart, cardiomyopathy or failing heart.

In a sixth more preferred embodiment, the compounds of the invention are considered useful for the treatment, prevention or alleviation of a cardiovascular disease. In a more preferred embodiment the cardiovascular disease is atherosclerosis, ischemia/reperfusion, hypertension, restenosis, arterial inflammation, myocardial ischaemia or ischaemic heart disease.

In a seventh more preferred embodiment, the β-keto-amide derivatives of the invention are considered useful for the treatment, prevention or alleviation of cardiac arrhythmia, atrial fibrillation and/or ventricular tachyarrhythmia.

In an eighth more preferred embodiment, the compounds of the invention are considered useful for obtaining preconditioning of the heart. Preconditioning, which includes ischemic preconditioning and myocardial preconditioning, describes short periods of ischemic events before initiation of a long lasting ischemia. The compounds of the invention are believed having an effect similar to preconditioning obtained by such ischemic events. Preconditioning protects against later tissue damage resulting from the long lasting ischemic events.

In a ninth more preferred embodiment, the β-keto-amide derivatives of the invention are considered useful for the treatment, prevention or alleviation of schizophrenia, depression or Parkinson's disease.

In a tenth more preferred embodiment, the compounds of the invention are considered useful for the treatment, prevention or alleviation of an obstructive or inflammatory airway disease. In a more preferred embodiment the the obstructive or inflammatory airway disease is an airway hyperreactivity, a pneumoconiosis such as aluminosis, anthracosis, asbestosis, chalicosis, ptilosis, siderosis, silicosis, tabacosis and byssinosis, a chronic obstructive pulmonary disease (COPD), bronchitis, excerbation of airways hyperreactivity or cystic fibrosis.

In its most preferred embodiment the obstructive airway disease is chronic obstructive pulmonary disease (COPD).

In an eleventh more preferred embodiment, the β-keto-amide derivatives of the invention are considered useful for the treatment, prevention or alleviation of a sexual dysfunction, incl. male sexual dysfunction and female sexual dysfunction, and incl. male erectile dysfunction.

In an even more preferred embodiment the β-keto-amide derivative of the invention may be co-administered with a phosphodiesterase inhibitor, in particular a phosphodiesterase 5 (PDE5) inhibitor, e.g. sildenafil, tadalafil, vardenafil and dipyridamole, or with an agent that potentiates endothelium-derived hyperpolarizing factor-mediated responses, in particular calcium dobesilate or similar 2,5-dihydroxybenzenesulfonate analogs.

In a most preferred embodiment the β-keto-amide derivative of the invention is used in a combination therapy together with sildenafil, tadalafil, vardenafil or calcium dobesilate.

In a twelfth more preferred embodiment, the acetamide derivatives of the invention are considered useful for the treatment, prevention or alleviation of ophthalmic angiogenesis related diseases, disorders or conditions, such as exudative macular degeneration, age-related macular degeneration (AMD), retinopathy, diabetic retinopathy, proliferative diabetic retinopathy, diabetic macular edema (DME), ischemic retinopathy (e.g. retinal vain or artery occlusion), retinopathy of prematurity, neovascular ocular hypertension, glaucoma and corneal neovascularization. In the context of this invention, "age-related macular degeneration" (AMD) includes dry AMD (non-exudative AMD) and wet AMD (exudative AMD). In a special embodiment, the invention relates to treatment, prevention or alleviation of wet AMD.

The acetamide derivatives of the invention are considered particular useful for the treatment of a disease, disorder or condition that is responsive to reduction of intraocular pressure, such as ocular hypertension, open-angle glaucoma, chronic open-angle glaucoma, angle-closure glaucoma and ciliary injection caused by angle-closure glaucoma.

It is at present contemplated that a suitable dosage of the active pharmaceutical ingredient (API) is within the range of from about 0.1 to about 1000 mg API per day, more preferred of from about 10 to about 500 mg API per day, most preferred of from about 30 to about 100 mg API per day, dependent, however, upon the exact mode of administration, the form in which it is administered, the indication considered, the subject and in particular the body weight of the subject involved, and further the preference and experience of the physician or veterinarian in charge.

Preferred β-keto-amide derivatives of the invention show a biological activity in the sub-micromolar and micromolar range, i.e. of from below 1 to about 100 μM.

## Pharmaceutical Compositions

In another aspect the invention provides novel pharmaceutical compositions comprising a therapeutically effective amount of a β-keto-amide derivative of the invention.

While a β-keto-amide derivative of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the β-keto-amide derivative of the invention together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in

dragé, in powder, or in liquid form, and parenteral administration, in particular cutaneous, subcutaneous, intramuscular, or intravenous injection. The pharmaceutical composition of the invention can be manufactured by any person skilled in the art, by use of standard methods and conventional techniques, appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day.

A satisfactory result can, in certain instances, be obtained at a dosage as low as 0.1 μg/kg i.v. and 1 μg/kg p.o. The upper limit of the dosage range is presently considered to be about 10 mg/kg i.v. and 100 mg/kg p.o. Preferred ranges are from about 0.1 μg/kg to about 10 mg/kg/day i.v., and from about 1 μg/kg to about 100 mg/kg/day p.o.

**Pharmaceutical Kits of Parts**

According to the invention there is also provided a kit of parts comprising at least two separate unit dosage forms (A) and (B):

(A) a β-keto-amide derivative of the invention; and
(B1) a phosphodiesterase inhibitor; or
(B2) an agent that potentiates endothelium-derived hyperpolarizing factor-mediated responses; and optionally
(C) instructions for the simultaneous, sequential or separate administration of the β-keto-amide derivative of A, and the phosphodiesterase inhibitor of B1, or an agent that potentiates endothelium-derived hyperpolarizing factor-mediated responses of B2, to a patient in need thereof.

In a more preferred embodiment the phosphodiesterase inhibitor for use according to the invention (B1) is a phosphodiesterase 5 (PDE5) inhibitor, and in an even more preferred embodiment the phosphodiesterase inhibitor for use according to the invention is sildenafil, tadalafil or vardenafil.

In another more preferred embodiment the agent that potentiates endothelium-derived hyperpolarizing factor-mediated responses for use according to the invention (B2) is calcium dobesilate.

The β-keto-amide derivative of the invention and the phosphodiesterase inhibitor or the agent that potentiates endothelium-derived hyperpolarizing factor-mediated responses for use according to the invention may preferably be provided in a form that is suitable for administration in conjunction with the other. This is intended to include instances where one or the other of two formulations may be administered (optionally repeatedly) prior to, after, and/or at the same time as administration with the other component.

Also, the β-keto-amide derivative of the invention and the phosphodiesterase inhibitor or the agent that potentiates endothelium-derived hyperpolarizing factor-mediated responses for use according to the invention may be administered in a combined form, or separately or separately and sequentially, wherein the sequential administration is close in time or remote in time. This may in particular include that two formulations are administered (optionally repeatedly) sufficiently closely in time for there to be a beneficial effect for the patient, that is greater over the course of the treatment of the relevant condition than if either of the two formulations are administered (optionally repeatedly) alone, in the absence of the other formulation, over the same course of treatment. Determination of whether a combination provides a greater beneficial effect in respect of, and over the course of treatment of, a particular condition, will depend upon the condition to be treated or prevented, but may be achieved routinely by the person skilled in the art.

When used in this context, the terms "administered simultaneously" and "administered at the same time as" include that individual doses of the positive allosteric nicotine receptor modulator and the cognitive enhancer are administered within 48 hours, e.g. 24 hours, of each other.

Bringing the two components into association with each other, includes that components (A) and (B) may be provided as separate formulations (i.e. independently of one another), which are subsequently brought together for use in conjunction with each other in combination therapy; or packaged and presented together as separate components of a "combination pack" for use in conjunction with each other in combination therapy.

**Methods of Therapy**

In another aspect the invention provides a method of treatment, prevention or alleviation of a disease, disorder

or condition of a living animal body, including a human, which disorder, disease or condition is responsive to modulation of an ion channel, and in particular a potassium channel or a chloride channel, which method comprises the step of administering to such a living animal body in need thereof, a therapeutically effective amount a compound capable of activating the potassium channel, or a pharmaceutically-acceptable addition salt thereof.

The preferred medical indications contemplated according to the invention are those stated above.

It is at present contemplated that a suitable dosage of the active pharmaceutical ingredient (API) is within the range of from about 0.1 to about 1000 mg API per day, more preferred of from about 1 to about 500 mg API per day, most preferred of from about 1 to about 100 mg API per day, dependent, however, upon the exact mode of administration, the form in which it is administered, the indication considered, the subject and in particular the body weight of the subject involved, and further the preference and experience of the physician or veterinarian in charge.

## BRIEF DESCRIPTION OF THE DRAWINGS

The present invention is further illustrated by reference to the accompanying drawing, in which Fig. 1A and 1B show the effect of Compound 4 (i.e. *N*-[5-Chloro-2-(1*H*-tetrazol-5-yl)-phenyl]-3-(4-chloro-3-trifluoromethyl-phenyl)-3-oxo-propionamide) on the voltage dependence of $BK_{ca}$ channels expressed in *Xenopus* Oocytes:

Fig. 1A shows conductance (μS) vs. membrane potential (mV) in the absence (Control) of Compound 4 and in the presence of 0.01 to 31.6 μM of Compound 4; and

Fig. 1B shows the concentration-response relationship for the left-shift of the $BK_{ca}$-activation curve; i.e. ΔV (mV) vs. log [c] (M). The calculated EC50 and $\Delta V_{max}$ values for Compound 4 are 1.8 μM and -123 mV, respectively.

## EXAMPLES

The invention is further illustrated with reference to the following examples, which are not intended to be in any way limiting to the scope of the invention as claimed.

### Example 1

### Preparatory Example

The compounds according to the invention may be easily prepared by conventional methods, as for example as described by Clayden J, Greeves N, Warren S and Wothers P: "Nucleophilic substitution at the carbonyl group"; Organic Chemistry (2001) Oxford University press, involving a condensation of the suitable β-ketonic esters with a substituted aniline in boiling xylene.

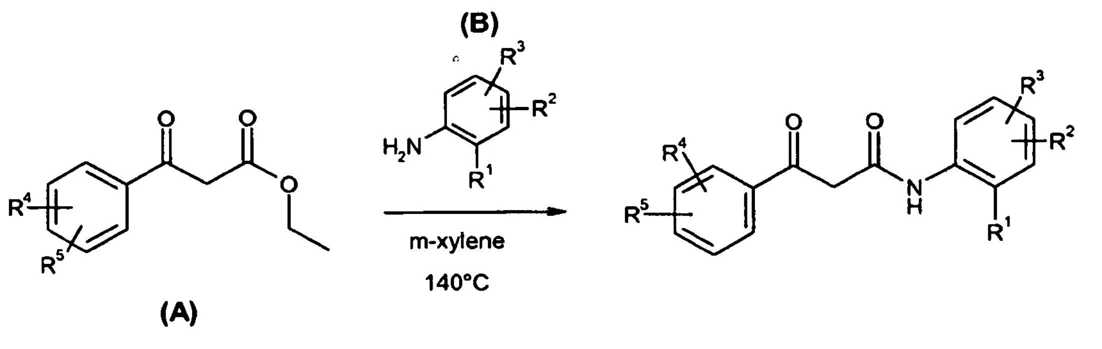


β-ketonic esters (**A**) are commercially-available or may be synthesised according to the well-known Claisen condensation between ketones and esters, e.g. as described by Cé/ine Mordant, Cristina Caño de Andrade, Ridha Touati, Virginie Ratovelomanana-Vidal, Bechir Ben Hassine, Jean-Pierre Genêt in Synthesis 2003 2405.

When the suitable anilines (**B**) were not commercially available they were synthesised either as described in e.g. WO 98/47879, in Valgeirsson et al.; Journal of Medicinal Chemistry 2004 47 (27) 6948-6957 or by palladium catalyzed Suzuki cross-coupling reaction between halogenated anilines and suitably-substituted arylboronic acids. In case the starting halogenated aniline is substituted by a ciano group in the ortho position, the Suzuki cross-coupling reaction is followed by the conversion of the cyano moiety to the correspondent tetrazolyl or 5-oxo-4,5-dihydro-[1,2,4]oxadiazol-3-yl derivative, as described in Valgeirsson et al. in Journal of Medicinal Chemistry 2004 47 (27) 6948-6957. As an example

of the synthetic experimental procedure for a non-commercial-aniline derivative B, the synthesis of the intermediate D is reported.

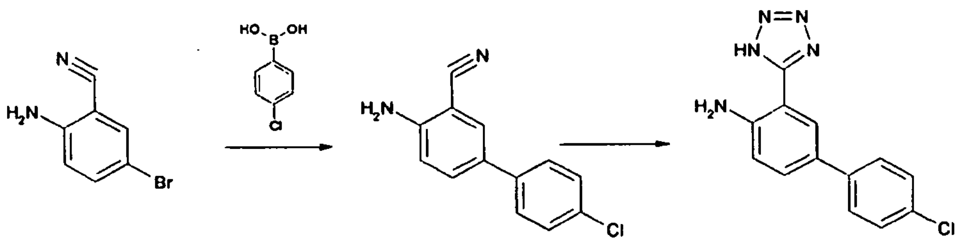

C

D

*4-Amino-4'-chloro-biphenyl-3-carbonitrile* (Intermediate compound) (**C**)

To a mixture of 2-amino-5-bromo-benzonitrile (5.5 g, 1 eq), 4-chlorobenzeneboronic acid (4.8 g, 1.1 eq), potassium carbonate (12.7 g, 3.3 eq), dimethoxy ethane (80 ml) and water (40 ml), bistriphenylphosphine palladium (II) chloride (0.2 g) is added. The resulting mixture is refluxed for 24 hours and then evaporated to dryness. The residue is purified by flash chromatography using DCM as eluent (5,32 g, 83% yield).

*4'-Chloro-3-(1H-tetrazol-5-yl)-biphenyl-4-ylamine* (Intermediate compound) (**D**)

A mixture of 4-amino-4'-chloro-biphenyl-3-carbonitrile (5.3 g, 1 eq), sodium azide (2.3 g, 1.5 eq), trethylamine hydrochloride (4.9 g, 1.5 eq) is suspended in 40 ml of toluene and heated (60˚C) overnight. To the reaction mixture, cooled to room temperature, water and 4M HCl are added, to afford the title compound as a white precipitate. This was collected by filtration (4.83 g, 77% yield) and used for the next step without further purification.

*3-(3,5-Bis-trifluoromethyl-phenyl)-N-[4-bromo-2-(1H-tetrazol-5-yl)-phenyl]-3-oxo-propionamide* (**1**)

A suspension of 3-(3,5-bis-trifluoromethyl-phenyl)-3-oxo-propionic acid ethyl ester (0.2 g) and 4-bromo-2-(1H-tetrazol-5-yl)-phenylamine (0.146 g, 1 eq) in m-xylene (20 ml) is refluxed for 3 hours. The resulting solution is evaporated to dryness and the residue is purified by crystallization from ethanol (0.25 g, ~80% yield). LC-ESI-HRMS of [M-H]- shows 519.984 Da. Calc. 519.984381 Da, dev. -0.7 ppm.

*3-(3,5-Bis-trifluoromethyl-phenyl)-N-[4'-chloro-3-(1H-tetrazol-5-yl)-biphenyl-4-yl]-3-oxo-propionamide* (**2**)

A suspension of 3-(3,5-bis-trifluoromethyl-phenyl)-3-oxo-propionic acid ethyl ester (0.2 g) and 4'-chloro-3-(1H-tetrazol-5-yl)-biphenyl-4-ylamine (0.166 g, 1 eq) in m-xylene (20 ml) is refluxed for 3 hours. The resulting solution is evaporated to dryness and the residue is purified by crystallization from acetonitrile (0.19 g, ~31% yield). LC-ESI-HRMS of [M+H]+ shows 554.0814 Da. Calc. 554.081846 Da, dev.

*3-(3,5-Bis-trifluoromethyl-phenyl)-3-oxo-N-[3-(1H-tetrazol-5-yl)-4'-trifluoromethoxy-biphenyl-4-yl]-propionamide* (**3**)

A suspension of 3-(3,5-bis-trifluoromethyl-phenyl)-3-oxo-propionic acid ethyl ester (0.2 g) and 3-(1H-tetrazol-5-yl)-4'-trifluoromethoxy-biphenyl-4-ylamine (0.196 g, 1 eq) in m-xylene (20 ml) is refluxed for 3 hours. The resulting solution is evaporated to dryness and the residue is purified by crystallization from ethanol (0.136 g, ~22% yield). LC-ESI-HRMS of [M+H]+ shows 604.1049 Da. Calc. 604.103117 Da, dev. 3 ppm.

*N-[5-Chloro-2-(1H-tetrazol-5-yl)-phenyl]-3-(4-chloro-3-trifluoromethyl-phenyl)-3-oxo-propionamide* (**4**)

A suspension of 3-(4-chloro-3-trifluoromethyl-phenyl)-3-oxo-propionic acid ethyl ester (0.1 g) and 5-chloro-2-(1H-tetrazol-5-yl)-phenylamine (0.068 g, 1 eq) in m-xylene (10 ml) is refluxed for 3 hours. The resulting solution is evaporated to dryness and the residue is purified by flash chromatography eluting with methanol / dichloromethane (0.41 g, ~26% yield). M.p. 207˚C. LC-ESI-HRMS of [M-H]- shows 442,0092 Da. Calc. 442,00854 Da, dev. 1,5 ppm.

**Example 2**

**BK Channel Activation**

In this example the BK channel opening activity of Compound 4 (i.e. *N*-[5-Chloro-2-(1*H*-tetrazol-5-yl)-phenyl]-3-(4-chloro-3-trifluoromethyl-phenyl)-3-oxo-propionamide) is determined using BK channels heterologously expressed in *Xenopus laevis* oocytes.

The electrical current through the BK channel is measured using conventional two-electrode voltage clamp. BK currents are activated by repeating ramp protocols. In brief, the membrane potential is continuously changed from -120 mV to +120 mV within 2 s. The threshold for BK activation is approximately +30 mV under control conditions. Compounds are applied for 100 s during which the ramp protocol is repeated 10 times with 10 s intervals. In between the ramp protocols the membrane potential is clamped at -80 mV. The first three compound applications are control blanks where the current level is allowed to stabilize. In the subsequent 8 applications increasing concentrations (0.01-31.6 $\mu$M) of compound is applied and a marked increase in the current level at depolarizing potentials is observed.

In order to evaluate the ability of the compounds to shift the BK activation curve towards lower membrane potentials, the BK current was transformed into conductance by using Ohm's law g = I ($E_{memb}$ - $E_{rev}$), where g is the conductance, I is the current, $E_{memb}$ is the membrane potential and $E_{rev}$ is the reversal potential. The extracellular solution for these experiments contained 2.5 mM $K^+$ and the intracellular $K^+$ concentration of an oocyte is estimated to be 100 mM. Under those conditions, Nernst equation predicts a reversal potential of $E_{rev}$= -93.2 mV. The control conductance level at a membrane potential of +100 mV was calculated, and the compound effect was evaluated as the potential difference, $\Delta$V, to the membrane potential at which the same conductance level was obtained in the presence of compound.

The concentration response curve for this potential difference was fitted to the sigmoidal logistic equation: $\Delta V = \Delta V_{max} /(1 + (EC_{50}/compound])^n)$, where $\Delta V_{max}$ represents the maximal left shift of the BK activation curve, $EC_{50}$ is the concentration causing a half maximal response, and n is the slope coefficient. The calculated EC50 and $\Delta V_{max}$ values for Compound **4** are 1.8 $\mu$M and -123 mV, respectively.

The results are presented in Figs. 1A and 1B.

**Example 3**

***In vitro* human erythrocyte chloride conductance**

In this example the chloride channel blocking activity of an acetamide derivative representative of the invention, i.e. Compound 3 (3-(3,5-Bis-trifluoromethylphenyl)-3-oxo-N-[3-(1H-tetrazol-5-yl)-4'-trifluoromethoxy-biphenyl-4-yl]-propionamide), has been determined.

All dose-response experiments were therefore performed by concomitant measurements of conductive netfluxes of $Cl^-$ ($J_{cl}$) and membrane potentials ($V_m$) in suspensions of erythrocytes as described by Bennekou et al. (Bennekou P and Christophersen P: Flux ratio of Valinomycin - Mediated K+ Fluxes across the Human Red Cell Membrane in the presence of the Protronophore CCCP; J. Membrane Biol. 1986 93 221-227).

The membrane Cl-conductances ($G_{cl}$) were calculated according to *Hodgkin et al.* (Hodgkin AL and Huxley AF: The components of membrane conductance in the giant axon of Loligo; J. Physiol. Lond. 1952 116 449-472) using the following equation:

$$G_{Cl} = \frac{F * J_{Cl}}{(V_m - E_{Cl})}$$

where F is the Faraday constant and $E_{cl}$ is the Nernst potential for the Cl-ion.

The $K_D$-value for Compound 3 was calculated as 0.21 $\mu$M.

## Claims

**1.** A $\beta$-keto-amide derivative of Formula I

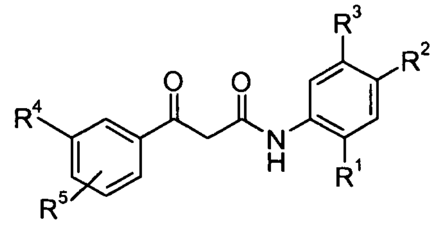

(I)

a stereoisomer or a mixture of its stereoisomers, or a pharmaceutically-acceptable addition salt thereof, wherein $R^1$ represents a tetrazolyl or a 5-oxo-4,5-dihydro-[1,2,4]oxadiazol-3-yl group; $R^2$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and
$R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy; or
$R^2$ represents hydrogen; and
$R^3$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and
$R^4$ and $R^5$, independently of each other, represent halo, trifluoromethyl, trifluoromethoxy or phenyl.

**2.** The β-keto-amide derivative of claim 1, or a pharmaceutically-acceptable addition salt thereof, wherein
$R^2$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and
$R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy; or
$R^2$ represents hydrogen; and
$R^3$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy.

**3.** The β-keto-amide derivative of claim 2, or a pharmaceutically-acceptable addition salt thereof, wherein
$R^2$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy; and
$R^3$ represents hydrogen, halo, trifluoromethyl, trifluoromethoxy, cyano, nitro, hydroxy or phenyl, which phenyl is optionally substituted one or more times with halo, trifluoromethyl, trifluoromethoxy and/or hydroxy.

**4.** The β-keto-amide derivative of claim 2, or a pharmaceutically-acceptable addition salt thereof, wherein
$R^2$ represents hydrogen; and
$R^3$ represents halo, trifluoromethyl, trifluoromethoxy, cyano, nitro, hydroxy or phenyl, which phenyl is optionally substituted one or more times with halo, trifluoromethyl, trifluoromethoxy and/or hydroxy.

**5.** The β-keto-amide derivative of any one of claims 1-4, or a pharmaceutically-acceptable addition salt thereof, wherein $R^4$ and $R^5$, independently of each other, represent halo or trifluoromethyl or trifluoromethoxy.

**6.** The β-keto-amide derivative of claim 1, which is
3-(3,5-Bis-trifluoromethyl-phenyl)-*N*-[4-bromo-2-(1*H*-tetrazol-5-yl)-phenyl]-3-oxo-propionamide;
3-(3,5-Bis-trifluoromethyl-phenyl)-*N*-[4'-chloro-3-(1*H*-tetrazol-5-yl)-biphenyl-4-yl]-3-oxo-propionamide;
3-(3,5-Bis-trifluoromethyl-phenyl)-3-oxo-*N*-[3-(1*H*-tetrazol-5-yl)-4'-trifluoromethoxy-biphenyl-4-yl]-propionamide; or
*N*-[5-Chloro-2-(1*H*-tetrazol-5-yl)-phenyl]-3-(4-chloro-3-trifluoromethylphenyl)-3-oxo-propionamide;
or a pharmaceutically-acceptable addition salt thereof.

**7.** A pharmaceutical composition comprising a therapeutically effective amount of the β-keto-amide derivative of any one of claims 1-6, or a pharmaceutically-acceptable addition salt thereof, or a prodrug thereof, together with one or more adjuvants, excipients, carriers and/or diluents.

**8.** Use of a β-keto-amide derivative of any one of claims 1-6, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of ion channels.

**9.** The use according to claim 8, wherein the disease, disorder or condition is a respiratory disease, epilepsy, convulsions, seizures, absence seizures, vascular spasms, coronary artery spasms, motor neuron diseases, myokymia, renal disorders, polycystic kidney disease, bladder hyperexcitability, bladder spasms, urinogenital disorders, urinary incontinence, bladder outflow obstruction, erectile dysfunction, gastrointestinal dysfunction, gastrointestinal hypomotility disorders, gastrointestinal motility insufficiency, postoperative ileus, constipation, gastroesophageal reflux disorder, secretory diarrhoea, an obstructive or inflammatory airway disease, ischaemia, cerebral ischaemia, ischaemic heart disease, angina pectoris, coronary heart disease, ataxia, traumatic brain injury, stroke, Parkinson's disease, bipolar disorder, psychosis, schizophrenia, autism, anxiety, mood disorders, depression, manic depression, psychotic disorders, dementia, learning deficiencies, age related memory loss, memory and attention deficits, Alzheimer's disease, amyotrophic lateral sclerosis (ALS), dysmenorrhea, narcolepsy, sleeping disorders, sleep apnea, Reynaud's disease, intermittent claudication, Sjogren's syndrome, xerostomia, arrhythmia, cardiovascular disorders, hypertension, myotonic dystrophy, myotonic muscle dystrophia, spasticity, xerostomi, diabetes Type II, hyperinsulinemia, premature labour, cancer, brain tumors, inflammatory bowel disease, irritable bowel syndrome, colitis, colitis Crohn, immune suppression, hearing loss, migraine, pain, neuropathic pain, inflammatory pain, trigeminal neuralgia, vision loss, rhinorrhoea, ocular hypertension (glaucoma), baldness, cardiac arrhythmia, atrial arrhythmia, ventricular arrhythmia, atrial fibrillation, ventricular fibrillation, tachyarrhythmia, atrial tachyarrhythmia, ventricular tachyarrhythmia, bradyarrhythmia, or any other abnormal rhythm, e.g. caused by myocardial ischaemia, myocardial infarction, cardiac hypertrophy or cardiomyopathy.

**10.** The β-keto-amide derivative of any one of claims 1-6, or a pharmaceutically-acceptable addition salt thereof, for use as a medicament.

**Patentansprüche**

**1.** β-Keto-amid-Derivat der Formel I

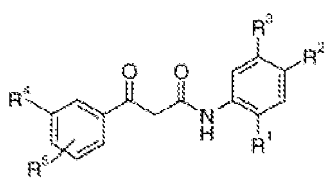

ein Stereoisomer oder eine Mischung von seinen Stereoisomeren oder ein pharmazeutisch verträgliches Additionssalz davon, worin

$R^1$ ein Tetrazolyl oder eine 5-Oxo-4,5-dihydro-[1,2,4]oxadiazol-3-yl-Gruppe bedeutet;

$R^2$ Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist;

$R^3$ Wasserstoff, Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist; oder

$R^2$ Wasserstoff bedeutet; und

$R^3$ Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist; und

$R^4$ und $R^5$ unabhängig voneinander Halogen, Trifluormethyl, Trifluormethoxy oder Phenyl bedeuten.

**2.** β-Keto-amid-Derivat nach Anspruch 1, oder ein pharmazeutisch verträgliches Additionssalz davon, worin

$R^2$ Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist; und

$R^3$ Wasserstoff, Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist; oder
$R^2$ Wasserstoff bedeutet; und
$R^3$ Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist.

**3.** β-Keto-amid-Derivat nach Anspruch 2, oder ein pharmazeutisch verträgliches Additionssalz davon, worin
$R^2$ Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist; und
$R^3$ Wasserstoff, Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist.

**4.** β-Keto-amid-Derivat nach Anspruch 2, oder ein pharmazeutisch verträgliches Additionssalz davon, worin
$R^2$ Wasserstoff bedeutet; und
$R^3$ Halogen, Trifluormethyl, Trifluormethoxy, Cyano, Nitro, Hydroxy oder Phenyl bedeutet, wobei dieses Phenyl gegebenenfalls einfach oder mehrfach mit Halogen, Trifluormethyl, Trifluormethoxy und/oder Hydroxy substituiert ist.

**5.** β-Keto-amid-Derivat nach einem der Ansprüche 1-4, oder ein pharmazeutisch verträgliches Additionssalz davon, worin $R^4$ und $R^5$, unabhängig voneinander, Halogen oder Trifluormethyl oder Trifluormethoxy bedeuten.

**6.** β-Keto-amid-Derivat nach Anspruch 1, welches 3-(3,5-Bis-trifluormethyl-phenyl)-*N*-[4-brom-2-(1*H*-tetrazol-5-yl)-phenyl]-3-oxo-propionamid;
3-(3,5-Bis-trifluormethyl-phenyl)-*N*-[4'-chlor-3-(1*H*-tetrazol-5-yl)-biphenyl-4-yl]-3-oxo-propionamid;
3-(3,5-Bis-trifluormethyl-phenyl)-3-oxo-*N*-[3-(1*H*-tetrazol-5-yl)-4'-trifluormethoxy-biphenyl-4-yl]-propionamid; oder
*N*-[5-Chlor-2-(1*H*-tetrazol-5-yl)-phenyl]-3-(4-chlor-3-trifluormethyl-phenyl)-3-oxo-propionamid ist;
oder ein pharmazeutisch verträgliches Additionssalz davon.

**7.** Pharmazeutische Zusammensetzung, umfassend eine therapeutisch wirksame Menge von dem β-Keto-amid-Derivat nach einem der Ansprüche 1-6, oder einem pharmazeutisch verträglichen Additionssalz davon, oder einem Prodrug davon, zusammen mit einem oder mehreren Adjuvantien, Exzipienten, Trägern und/oder Verdünnungsmitteln.

**8.** Verwendung von einem β-Keto-amid-Derivat nach einem der Ansprüche 1-6, oder einem pharmazeutisch verträglichen Additionssalz davon, für die Herstellung einer pharmazeutischen Zusammensetzung / eines Medikaments für die Behandlung, Verhütung oder Linderung einer Krankheit oder einer Störung oder eines Zustands eines Säugers, einschließlich eines Menschen, wobei die Krankheit, die Störung oder der Zustand auf die Modulation von Ionenkanälen anspricht.

**9.** Verwendung nach Anspruch 8, wobei es sich bei der Krankheit, der Störung oder dem Zustand um eine Atemwegserkrankung, Epilepsie, Krämpfe, Anfälle, Petit-mal-Epilepsie, Gefäßspasmen, Koronararterienspasmen, Motoneuronerkrankungen, Myokymie, Nierenerkrankungen, polyzystische Nierenerkrankung, Blasenübererregbarkeit, Blasenspasmen, urogenitale Störungen, Harninkontinenz, Blasenauslassobstruktion, erektile Dysfunktion, gastrointestinale Dysfunktion, gastrointestinale Hypomotilitätsstörungen, gastrointestinale Motilitätsinsuffizienz, postoperativen Ileus, Verstopfung, gastroösophageale Refluxerkrankung, sekretorische Diarrhoe, eine obstruktive oder entzündliche Erkrankung der Luftwege, Ischämie, zerebrale Ischämie, ischämische Herzerkrankung, Angina pectoris, koronare Herzerkrankung, Ataxie, traumatische Hirnverletzung, Schlaganfall, die Parkinson-Krankheit, eine bipolare Störung, Psychose, Schizophrenie, Autismus, Angst, affektive Psychosen, eine Depression, manische Depression, psychotische Störungen, eine Demenz, Lerndefizite, altersbedingten Gedächtnisverlust, Gedächtnis- und Aufmerksamkeitsdefizite, die Alzheimer-Krankheit, amyotrophe Lateralsklerose (ALS), Dysmenorrhoe, Narkolepsie, Schlafstörungen, Schlafapnoe, die Reynaud-Krankheit, intermittierendes Hinken,
das Sjögren-Syndrom, Xerostomie, Arrhythmie, kardiovaskuläre Erkrankungen, Hochdruck, myotone Dystrophie, myotone Muskeldystrophie, Spastizität, Xerostomie, Diabetes Typ II, Hyperinsulinämie, eine Frühgeburt, Krebs, Hirntumore, eine entzündliche Darmerkrankung, das Reizdarmsyndrom, Kolitis, Kolitis Crohn, eine Immunsuppression, einen Hörverlust, Migräne, Schmerzen, neuropathische Schmerzen, entzündliche Schmerzen, Trigeminusneuralgie, einen Verlust des Sehvermögens, Rhinorrhoe, Augenhochdruck (Glaucom), Kahlheit, kardiale Arrhythmie, atriale Arrhythmie, ventrikuläre Arrhythmie, Vorhofflimmern, Kammerflimmern, Tachyarrhythmie, atriale Tachyarrhythmie, ventrikuläre Tachyarrhythmie, Bradyarrhythmie, oder irgendeinen anderen abnormalen Rhythmus, z. B. verursacht durch myokardiale Ischämie, Myokardinfarkt, kardiale Hypertrophie oder Kardiomyopathie handelt.

**10.** β-Keto-amid-Derivat nach einem der Ansprüche 1-6, oder ein pharmazeutisch verträgliches Additionssalz davon, zur Verwendung als ein Medikament.

## Revendications

**1.** Dérivé de β-céto-amide de formule I

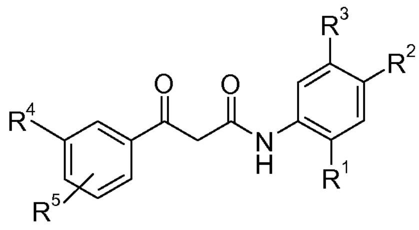

(I)

un stéréo-isomère ou un mélange de ses stéréo-isomères, ou un sel d'addition pharmaceutiquement acceptable de celui-ci, dans lequel
$R^1$ représente un tétrazolyle ou un groupe 5-oxo-4,5-dihydro-[1,2,4]oxadiazol-3-yle ;
$R^2$ représente un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy ; et
$R^3$ représente un hydrogène, un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy ; ou
$R^2$ représente un hydrogène ; et
$R^3$ représente un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy ; et
$R^4$ et $R^5$, indépendamment l'un de l'autre, représentent un halogéno, un trifluorométhyle, un trifluorométhoxy ou un phényle.

**2.** Dérivé de β-céto-amide selon la revendication 1, ou un sel d'addition pharmaceutiquement acceptable de celui-ci, dans lequel
$R^2$ représente un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy ; et
$R^3$ représente un hydrogène, un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy ; ou
$R^2$ représente un hydrogène ; et
$R^3$ représente un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy.

**3.** Dérivé de β-céto-amide selon la revendication 2, ou un sel d'addition pharmaceutiquement acceptable de celui-ci, dans lequel
$R^2$ représente un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy ; et
$R^3$ représente un hydrogène, un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy.

**4.** Dérivé de β-céto-amide selon la revendication 2, ou un sel d'addition pharmaceutiquement acceptable de celui-ci, dans lequel
$R^2$ représente un hydrogène ; et
$R^3$ représente un halogéno, un trifluorométhyle, un trifluorométhoxy, un cyano, un nitro, un hydroxy ou un phényle, lequel phényle est facultativement substitué une ou plusieurs fois par un halogéno, un trifluorométhyle, un trifluorométhoxy et/ou un hydroxy.

**5.** Dérivé de β-céto-amide selon l'une quelconque des revendications 1 à 4, ou un sel d'addition pharmaceutiquement acceptable de celui-ci, dans lequel $R^4$ et $R^5$, indépendamment l'un de l'autre, représentent un halogéno ou un trifluorométhyle ou un trifluorométhoxy.

**6.** Dérivé de β-céto-amide selon la revendication 1, qui est
le 3-(3,5-bis-trifluorométhyl-phényl)-*N*-[4-bromo-2-(1*H*-tétrazol-5-yl)-phényl]-3-oxo-propionamide ;
le 3-(3,5-bis-trifluorométhyl-phényl)-*N*-[4'-chloro-3-(1*H*-tétrazol-5-yl)-biphényl-4-yl]-3-oxo-propionamide ;
le 3-(3,5-bis-trifluorométhyl-phényl)-3-oxo-*N*-[3-(1*H*-tétrazol-5-yl)-4'-trifluorométhoxy-biphényl-4-yl]-propionamide ;
ou
le *N*-[5-chloro-2-(1*H*-tétrazol-5-yl)-phényl]-3-(4-chloro-3-trifluorométhyl-phényl)-3-oxo-propionamide ;
ou un sel d'addition pharmaceutiquement acceptable de celui-ci.

**7.** Composition pharmaceutique comprenant une quantité thérapeutiquement efficace du dérivé de β-céto-amide selon l'une quelconque des revendications 1 à 6, ou d'un sel d'addition pharmaceutiquement acceptable de celui-ci, ou d'un prodrogue de celui-ci, conjointement avec un ou plusieurs adjuvants, excipients, supports et/ou diluants.

**8.** Utilisation d'un dérivé de β-céto-amide selon l'une quelconque des revendications 1 à 6, ou d'un sel d'addition pharmaceutiquement acceptable de celui-ci, pour la fabrication d'un(e) composition pharmaceutique/médicament pour le traitement, la prévention ou l'atténuation d'une maladie ou d'un trouble ou d'une affection d'un mammifère, incluant un être humain, laquelle maladie, lequel trouble ou laquelle affection réagit à une modulation des canaux ioniques.

**9.** Utilisation selon la revendication 8, dans laquelle la maladie, le trouble ou l'affection est une maladie respiratoire, une épilepsie, des convulsions, des crises d'épilepsie, des absences, des spasmes vasculaires, des spasmes des artères coronariennes, des maladies des motoneurones, une myokymie, des troubles rénaux, une maladie polykystique du rein, une hyperexcitabilité de la vessie, des spasmes de la vessie, des troubles génito-urinaires, une incontinence urinaire, une obstruction de l'écoulement de la vessie, une dysérection, un dysfonctionnement gastro-intestinal, des troubles d'hypomotilité gastro-intestinale, une insuffisance de motilité gastro-intestinale, une occlusion intestinale postopératoire, une constipation, un trouble de reflux gastro-oesophagien, une diarrhée sécrétoire, une maladie des voies aériennes obstructive ou inflammatoire, une ischémie, une ischémie cérébrale, une cardiopathie ischémique, une angine de poitrine, une maladie coronarienne, une ataxie, une lésion cérébrale traumatique, une attaque, une maladie de Parkinson, un trouble bipolaire, une psychose, une schizophrénie, un autisme, une anxiété, des troubles de l'humeur, une dépression, une manie-dépression, des troubles psychotiques, une démence, des déficiences d'apprentissage, une perte de mémoire liée à l'âge, des déficiences de la mémoire et de l'attention, une maladie d'Alzheimer, une sclérose latérale amyotrophique (SLA), une dysménorrhée, une narcolepsie, des troubles du sommeil, une apnée du sommeil, une maladie de Reynaud, une claudication intermittente, un syndrome de Sjogren, une xérostomie, une arythmie, des troubles cardio-vasculaires, une hypertension, une dystrophie myotonique, une dystrophie musculaire myotonique, une spasticité, une xérostomie, un diabète de type II, une hyperinsulinémie, un travail prématuré, un cancer, des tumeurs cérébrales, une maladie intestinale inflammatoire, un syndrome du côlon irritable, une colite, une maladie de Crohn, une suppression immunitaire, une perte d'audition, une migraine, une douleur, une douleur neuropathique, une douleur inflammatoire, une névralgie du trijumeau, une perte de vision, une rhinorrhée, une hypertension oculaire (glaucome), une calvitie, une arythmie cardiaque, une arythmie atriale, une arythmie ventriculaire, une fibrillation atriale, une fibrillation ventriculaire, une tachyarythmie, une tachyarythmie atriale, une tachyarythmie ventriculaire, une bradyarythmie, ou tout autre rythme anormal, par exemple provoqué par une ischémie myocardique, un infarctus du myocarde, une hypertrophie cardiaque ou une

cardiomyopathie.

10. Dérivé de β-céto-amide selon l'une quelconque des revendications 1 à 6, ou un sel d'addition pharmaceutiquement acceptable de celui-ci, pour une utilisation en tant que médicament.

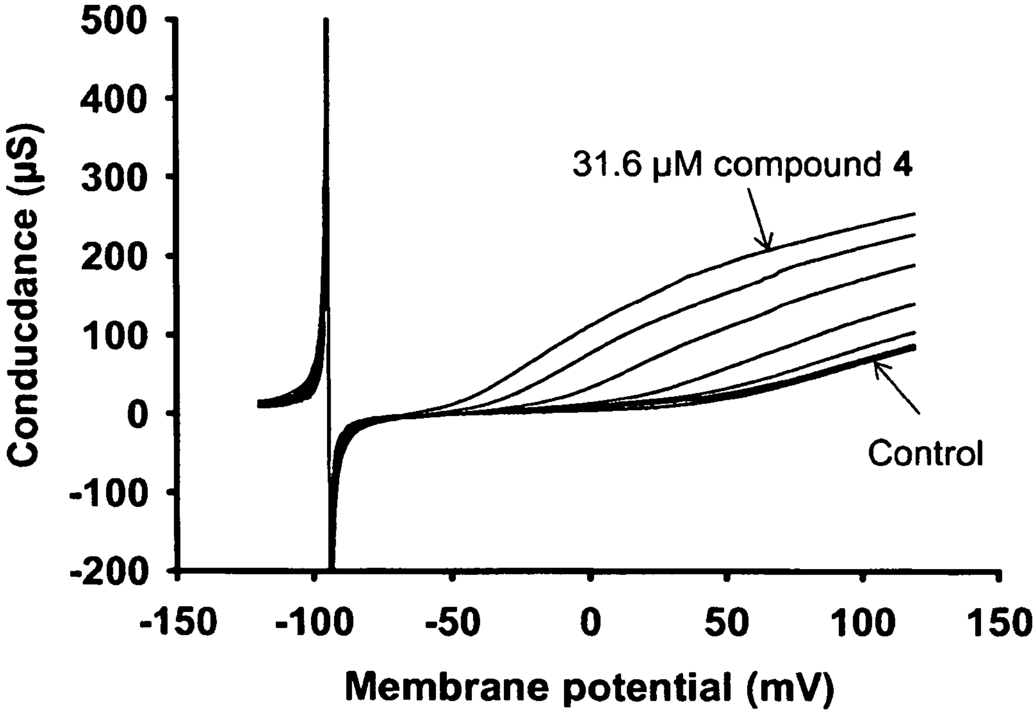
500
400
300
200
100
0
-100
-200
Conducdance (µS)
31.6 µM compound 4
Control
-150
-100
-50
0
50
100
150
Membrane potential (mV)

Fig. 1A

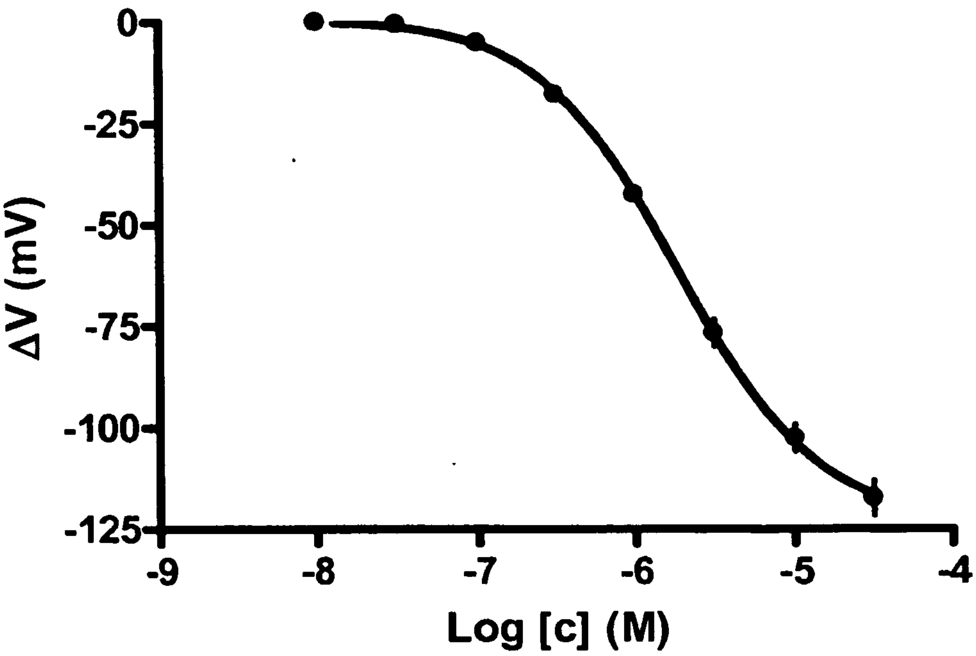


Fig. 1B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9847879 A


### Non-patent literature cited in the description


- **Jaques J ; Collet A ; Wilen S.** Enantiomers, Racemates, and Resolutions. John Wiley and Sons, 1981
- Remington's Pharmaceutical Sciences. Maack Publishing Co,
- Nucleophilic substitution at the carbonyl group. **Clayden J ; Greeves N ; Warren S ; Wothers P.** Organic Chemistry. Oxford University press, 2001
- **Cé/ine Mordant ; Cristina Caño de Andrade ; Ridha Touati ; Virginie Ratovelomanana-Vidal ; Bechir Ben Hassine ; Jean-Pierre Genêt.** *Synthesis,* 2003, 2405
- **Valgeirsson et al.** *Journal of Medicinal Chemistry,* 2004, vol. 47 (27), 6948-6957
- **Bennekou P ; Christophersen P.** Flux ratio of Valinomycin - Mediated K+ Fluxes across the Human Red Cell Membrane in the presence of the Protronophore CCCP. *J. Membrane Biol.,* 1986, vol. 93, 221-227
- **Hodgkin AL ; Huxley AF.** The components of membrane conductance in the giant axon of Loligo. *J. Physiol. Lond.,* 1952, vol. 116, 449-472